Europäisches Patentamt

European Patent Office

Office européen des brevets

(19)

(11) Publication number: **0 314 863 B1**

# EUROPEAN PATENT SPECIFICATION

(45) Date of publication of patent specification: **07.12.94**

(51) Int. Cl.5: **A61K 37/02**, A61K 39/395, //C12P21/00

(21) Application number: **88106902.5**

(22) Date of filing: **29.04.88**

(54) **Use of ICAM-1 or its functional derivatives for the treatment of non-specific inflammation.**

(30) Priority: **02.11.87 US 115797**

(43) Date of publication of application:
**10.05.89 Bulletin 89/19**

(45) Publication of the grant of the patent:
**07.12.94 Bulletin 94/49**

(84) Designated Contracting States:
**AT BE CH DE ES FR GB GR IT LI LU NL SE**

(56) References cited:

**THE JOURNAL OF IMMUNOLOGY, vol. 137, no. 4, 15th August 1986, pages 1270-1274, The American Association of Immunologists, US; R. ROTHLEIN et al.: "A human intercellular adhesion molecule (ICAM-1) distinct from LFA-1"**

**BIOLOGICAL ABSTRACTS/RRM, vol. 33, 1987, no. 37528; D.C. ANDERSON et al.:"Leukocyte adhesion deficiency an inherited defect in the MAC-1 LFA-1 A PL50 95 glycoproteins".**

(73) Proprietor: **BAYLOR COLLEGE OF MEDICINE**
**One Baylor Plaza**
**Houston, TX 77030 (US)**

Proprietor: **Boehringer Ingelheim Pharmaceuticals Inc.**
**90 East Ridge**
**P.O. Box 368**
**Ridgefield Connecticut 06877 (US)**

(72) Inventor: **Anderson, Donald Carroll**
**13811 Wilde Forest**
**Sugarland Texas 77478 (US)**
Inventor: **Rothlein, Robert**
**32 Tamanny Trail**
**Danbury Connecticut 06811 (US)**
Inventor: **Smith, Clinton Wayne**
**13927 Wilde Forest**
**Sugarland**
**Texas 77478 (US)**
Inventor: **Barton, Randall Wilbur**
**4 Fable Lane**
**Farmington**
**Connecticut 06032 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

THE JOURNAL OF IMMUNOLOGY, vol. 137, no. 1, 1st July 1986, The AmericanAssociation of Immunologists, US; M.L. DUSTIN et al.: "Induction by IL 1 andinterferon-mu: tissue distribution, biochemistry, and function of a naturaladherencce molecule (ICAM-1)"

(74) Representative: **Laudien, Dieter, Dr. et al Boehringer Ingelheim Zentrale GmbH ZA Patente Postfach 200 D-55216 Ingelheim am Rhein (DE)**

**Description**

The present invention relates to the use of intercellular adhesion molecules such as ICAM-1, antagonists capable of binding to ICAM-1, or ICAM-1-derived antagonists capable of binding to the natural binding partner of ICAM-1, to inhibit intercellular adhesion of cells of granulocyte or macrophage lineage.

Granulocytes must be able to adhere to cellular substrates in order for an inflammatory response to occur. This fact has become evident from two converging lines of research.

The first line of research involves studies of leukocyte membrane proteins (Wallis, W.J., et al., J. Immunol. 135:2323-2330 (1985); Mentzer, S.J., et al., J. Cell. Physiol. 126:285-290 (1986); Haskard, D.O., et al., J. Immunol. 137:2901-2906 (1986); Harlan, J.M., et al., Blood 66:167-178 (1985)). Of particular importance to the process of cellular adhesion is a family of leukocyte membrane proteins known as the "CD18" family or complex. This family consists of three heterodimers (known as "Mac-1," "LFA-1," and "P150,90"), all of which share a common subunit (known as the $\beta$ subunit) and a unique subunit (known as the $\alpha$ subunit) (Springer, T.A., et al., Immunol. Rev. 68:111-135 (1982); Springer, T., et al., Fed. Proc. 44:2660-2663 (1985); Keizer, G., et al., Eur. J. Immunol. 15:1142-1147 (1985); Sanchez-Madrid, F., et al., J. Exper. Med. 158:1785-1803 (1983)). Mac-1 is present on macrophages, granulocytes and other leukocytes.

Monoclonal antibodies against the CD18 family of leukocyte membrane proteins, by acting as antagonists of these proteins, inhibit a multitude of leukocyte adhesion dependent events in vitro. This includes the ability of granulocytes to aggregate in reponse to appropriate stimuli, the ability of granulocytes to attach to protein coated plastic, the ability of granulocytes to migrate in 2-dimensional agarose assays, and the ability of granulocytes to attach to endothelial cells.

The second line of research results from studies involving individuals, who, due to an inherited flaw in the gene encoding for the common subunit of the CD18 family of leukocyte adhesion molecules, are unable to express any of these adhesion molecules on the surfaces of their cells. Such individuals are said to suffer from "leukocyte adherence deficiency disease" ("LAD") (Anderson, D.C., et al., Fed. Proc. 44:2671-2677 (1985); Anderson, D.C., et al., J. Infect. Dis. 152:668-689 (1985)). Characteristic features of LAD patients include necrotic soft tissue lesions, impaired pus formation and wound healing, as well as abnormalities of adhesion-dependent leukocyte functions in vitro. Granulocytes from these LAD patients behave in the same defective manner in vitro as do their normal counterparts in the presence of anti-CD18 monoclonal antibody. That is, they are unable to perform adhesion related functions such as aggregation or attachment to endothelial cells. More importantly, however, is the observation that these patients are unable to mount a normal inflammatory response because of the inability of their granulocytes to attach to cellular substrates. Most remarkable is the observation that granulocytes from these LAD patients are unable to get to sites of inflammation such as skin infections due to their inability to attach to the endothelial cells in the blood vessels near the inflammation lesions. Such attachment is a necessary step for extravasation.

The molecules on the endothelium to which the granulocyte's adhesion molecules attach has, thus far, not been identified. The value of identifying the natural ligand of Mac-1 is apparent when one considers that the molecule itself or an antagonist of such a molecule may inhibit the interaction of granulocytes with endothelium and thus mimic the LAD patients which, in cases where the inflammatory response is detrimental to the individual, is a desirable quality.

Thus, in summary, the ability of granulocytes to maintain the health and viability of an animal requires that they be capable of adhering to other cells (such as endothelial cells). Granulocyte-endothelial cell adherence has been found to require cell-cell contacts which involve specific receptor molecules present on the granulocyte cell surface. These receptors enable the leukocyte to adhere to other leukocytes or to endothelial, and other non-vascular cells. The natural binding partner of these receptor molecules is unknown.

The cell surface receptor molecules of leukocytes have been found to be highly related to one another. Humans whose leukocytes lack these cell surface receptor molecules exhibit chronic and recurring infections, as well as other clinical symptoms. Inflammation reactions are mitigated when leukocytes are unable to adhere in a normal fashion due to the lack of functional adhesion molecules of the CD18 complex. Because leukocyte adhesion is involved in the process through which tissue inflammation arises, an understanding of the process of leukocyte adhesion is of significant value in defining a treatment for non-specific inflammation.

The invention relates to the use of ICAM-1 or a functional derivative thereof, in the manufacture of a pharmaceutical composition for the treatment of inflammation resulting from a response of the non-specific defense system in a mammalian subject. Said inflammation is associated with a condition selected from the group consisting of: adult respiratory distress syndrome; multiple organ injury syndrome secondary to septicemia; multiple organ injury syndrome secondary to trauma; reperfusion injury of myocardial or other tissues; acute glomerulonephritis; reactive arthritis; dermatosis with acute inflammatory components; acute purulent meningitis or other central nervous system inflammatory disorders; thermal injury; hemodialysis; leukapheresis; ulcerative colitis; Crohn's disease; necrotizing enterocolitis; granulocyte transfusion associated syndrome; and cytokine-induced toxicity.

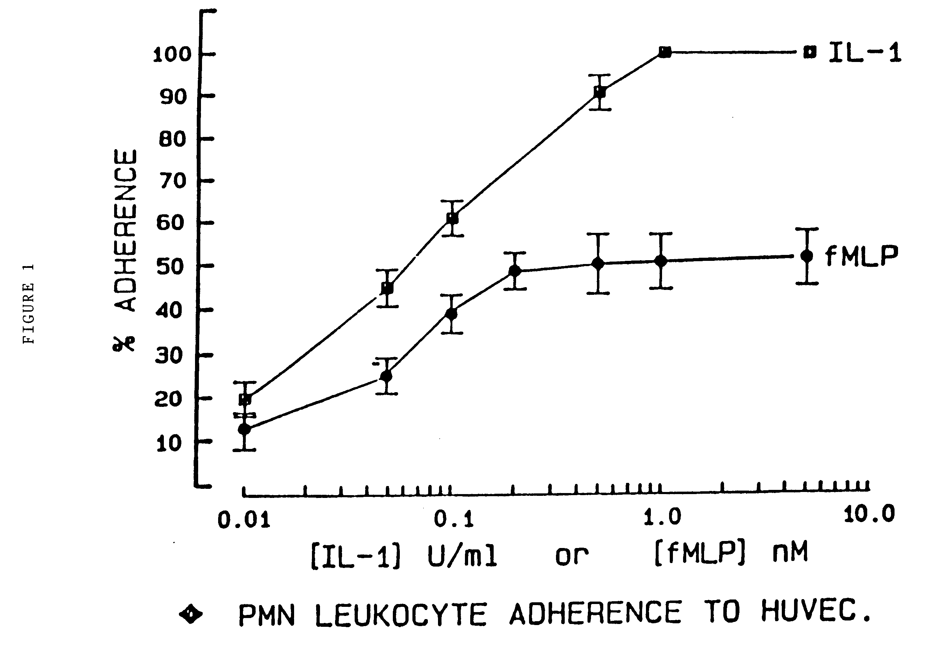

Figure 1 shows the effects of interleukin-1 (IL-1) and f-met-leu-phe (fMLP) on adherence of human peripheral blood polymorphonuclear lymphocytes (PMNL) to human umbilical cord vein endothelial cells (HUVEC). Effect of IL-1 was assessed in the following manner. HUVEC were incubated for 4 hr, 37°C, in the presence of the indicated concentrations of IL-1 and then washed by dipping 3 times in two exchanges of phosphate buffered saline (PBS). The adherence of unstimulated PMNL suspended in PBS was then determined using a visual assay performed at room temperature. The effect of fMLP was determined by suspending PMNL in the indicated concentrations of fMLP and incubating for 15 min at room temperature before determining adherence to unstimulated HUVEC using a visual assay performed at room temperature. Error bars, ±s.d.; n, 4; p<0.01 for all points beyond 0.05 nM fMLP and 0.01 U/ml IL-1.

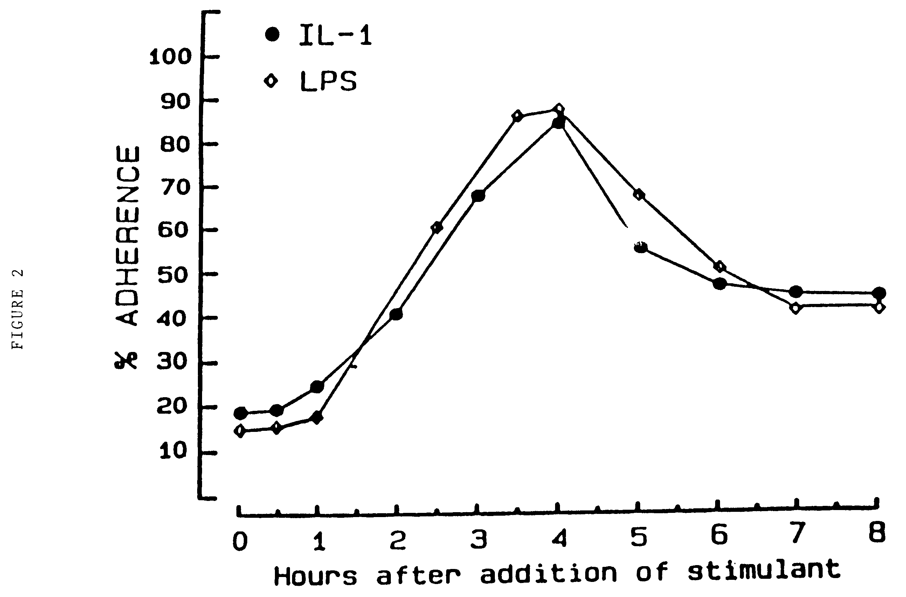

Figure 2 shows a comparison of the time courses for IL-1 and LPS stimulation of HUVEC adhesiveness. HUVEC monolayers were incubated at 37°C, in the presence of either LPS (10 ng/ml) or IL-1 (0.05 U/ml), then washed by dipping 3 times in two changes of PBS. Adherence of unstimulated PMNL suspended in PBS was assessed using a visual assay. Each point is the mean of three determinations. One standard deviation at each point was not greater than 10% of the mean.

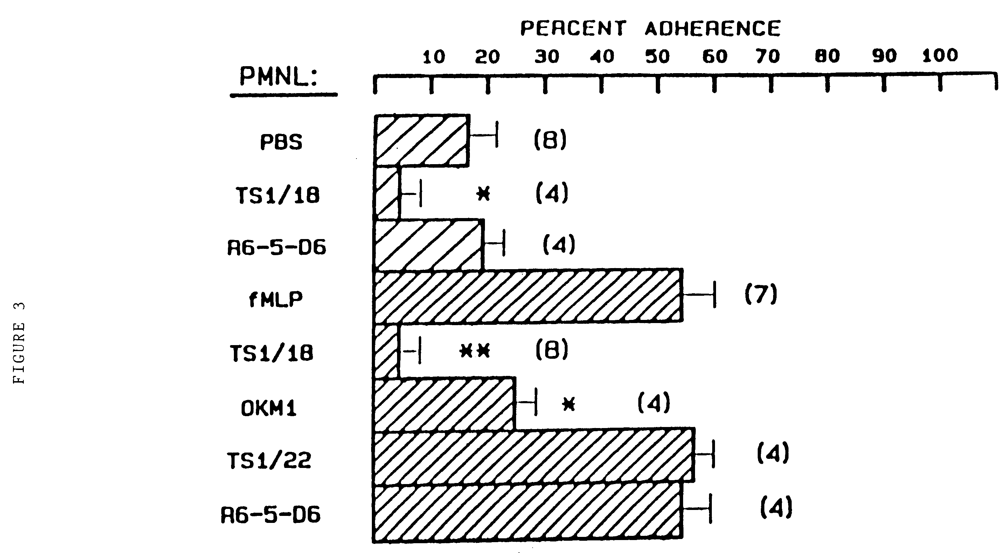

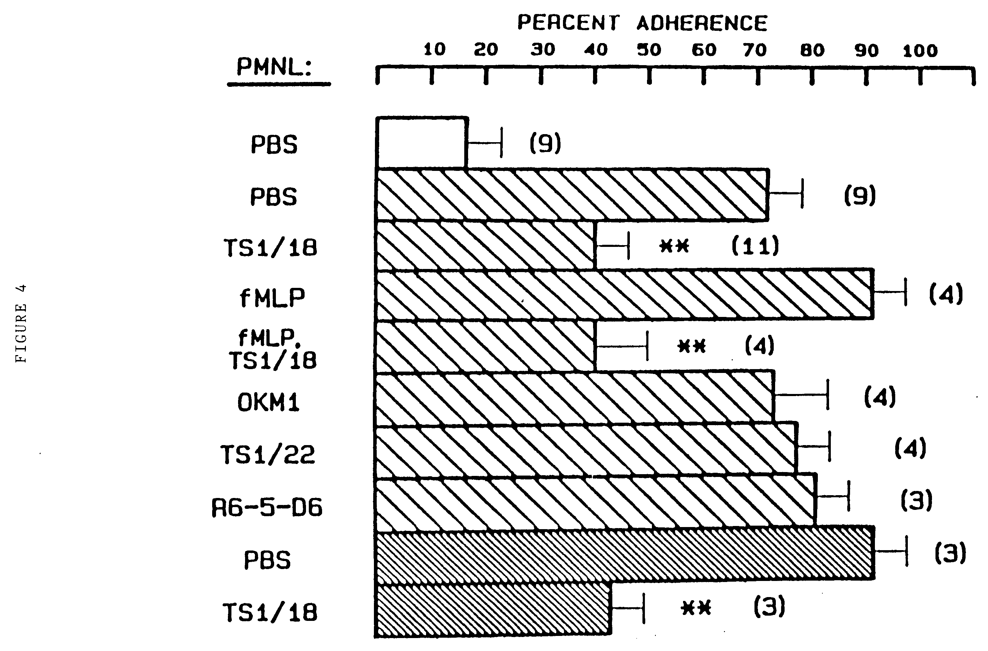

Figure 3 shows the effects of monoclonal antibodies on the adherence of human PMNL to unstimulated HUVEC monolayers. PMNL were preincubated for 15 min at room temperature in PBS (coarse hatched bars) with and without the indicated monoclonal antibodies or in 0.5 nM fMLP (fine hatched bars) with and without the indicated monoclonal antibodies before adherence to unstimulated HUVEC monolayers was assessed using a visual adherence assay performed at room temperature. TS1/18, 1:400 dilution of ascites; R6-5-D6, 1:400; dilution of ascites; fMLP, 0.5 nM; OKM1, 1:200 dilution of ascites; TS1/22, 1:200 dilution of ascites. ( ), number of separate experiments; error bars, ±s.d.; *, p<0.01; **, p<0.001.

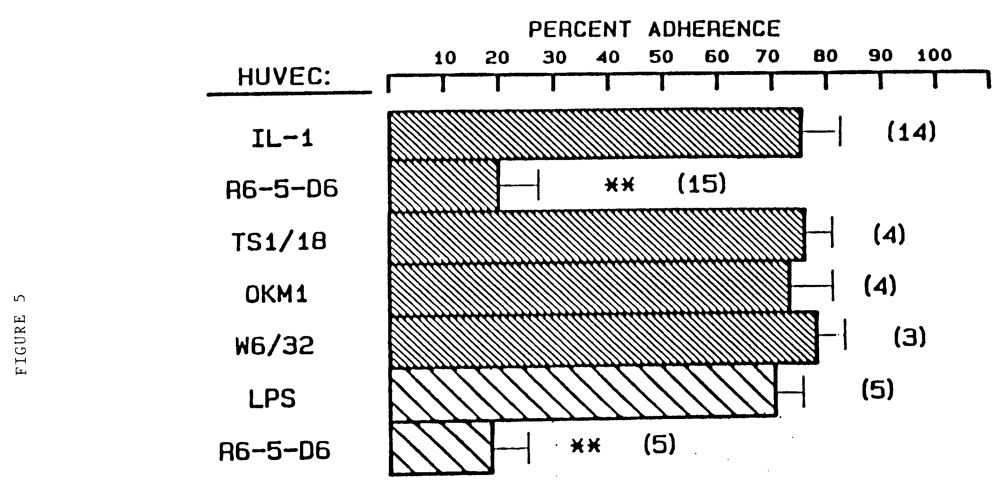

Figure 4 shows the effects of monoclonal antibodies on the adherence of human PMNL to lipopolysaccharide (LPS) and IL-1 stimulated HUVEC monolayers. PMNL were preincubated for 15 min at room temperature in the indicated reagents before adherence to HUVEC monolayers was assessed using a visual adherence assay performed at room temperature. HUVEC were incubated without (open bars) or with 10 ng/ml LPS (coarse hatched bars) or 0.5 U/ml IL-1 (fine hatched bars) for four hours at 37°C prior to use in the adherence assay. TS1/18, 1:400 dilution of ascites; R6-5-D6, 1:200 dilution of ascites, fMLP, 0.5 nM; OKM1, 1:200 dilution of ascites; TS1/22, 1:;200 dilution of ascites. ( ), number of separate experiments; error bars, ±s.d.; **, p<0.001.

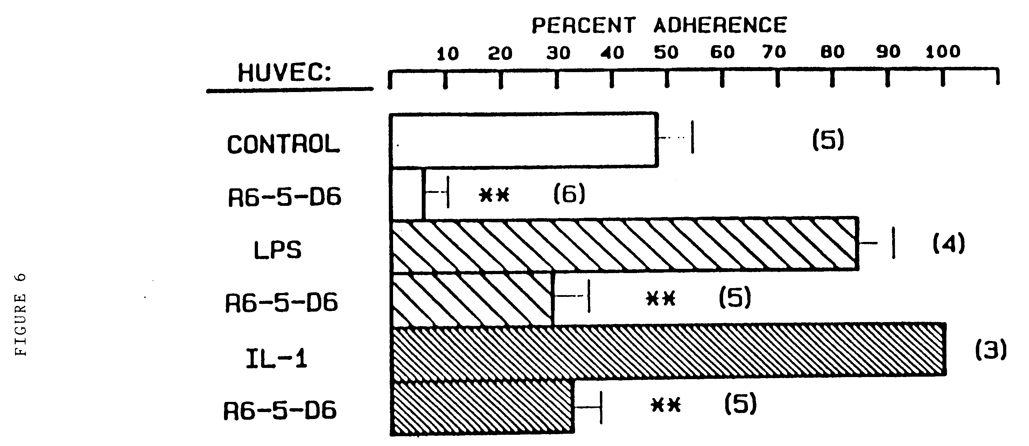

Figure 5 shows the effects of monoclonal antibodies on IL-1 and LPS enhanced HUVEC adhesiveness. HUVEC were incubated for 4 hr, 37°C, in the presence of 0.5 U/ml IL-1 (fine hatched bars) or 10 ng/ml LPS (coarse hatched bars), washed by dipping 3 times in two changes of PBS and then exposed to the indicated monoclonal antibodies of 15 min at 37°C, washed again and inserted in adherence chambers. Adherence of unstimulated human PMNL was assessed using a visual assay performed at room temperature. TS1/18, 1:400 dilution of ascites; R6-5-D6, 1:400 dilution of ascites; OKM1, 1:200 dilution of ascites; W6/32, 1:200 ascites. ( ), number of separate experiments; error bars ± 1 s.d.; **, p<0.001.

Figure 6 shows the effects of monoclonal antibody, R6-5-D6, on fMLP enhanced PMNL adherence to HUVEC. HUVEC were incubated without (open bars) or with 10 ng/ml LPS (coarse hatched bars) or 0.5 U/ml IL-1 (fine hatched bars) for 4 hr at 37°C, washed by dipping 3 times in two changes of PBS and then exposed to R6-5-D6 (1:400 dilution of ascites) for 15 min in 37°C. After washing, the adherence of fMLP (0.5 nM) preincubated (15 min, room temperature) PMNL was assessed using a visual adherence assay performed at room temperature. R6-5-D6, 1:400 dilution of ascites; fMLP, 0.5 nM; ( ), number of separate experiments; error bars, ± s.d.; **, p<0.001.

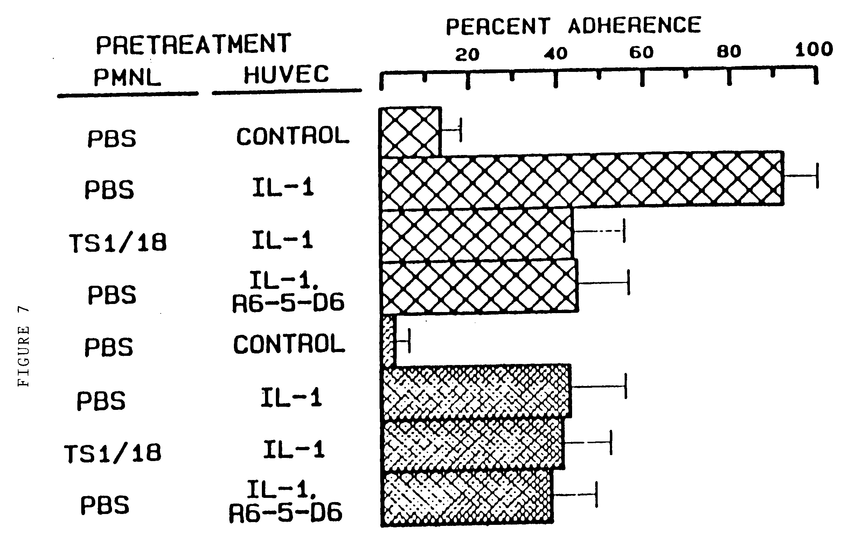

Figure 7 shows the adherence of CD18 deficient PMNL to HUVEC. Effects of monoclonal antibodies R6-5-D6 and TS1/18. HUVEC were incubated with and without IL-1 (0.5 U/ml) for 4 hr, 37°C, washed by dipping 3 times in two changes of PBS and then exposed to R6-5-D6 (1:400 dilution of ascites) for 15 min at 37°C. After washing, the adherence of normal (coarse grid bars) or deficient PMNL (fine grid bars) pretreated in PBS or TS1/18 (1:400 dilution of ascites) was assessed using a visual adherence assay performed at room temperature. Results of 4 separate experiments with PMNL from 2 LAD patients. Error bars, ± 1 s.d.

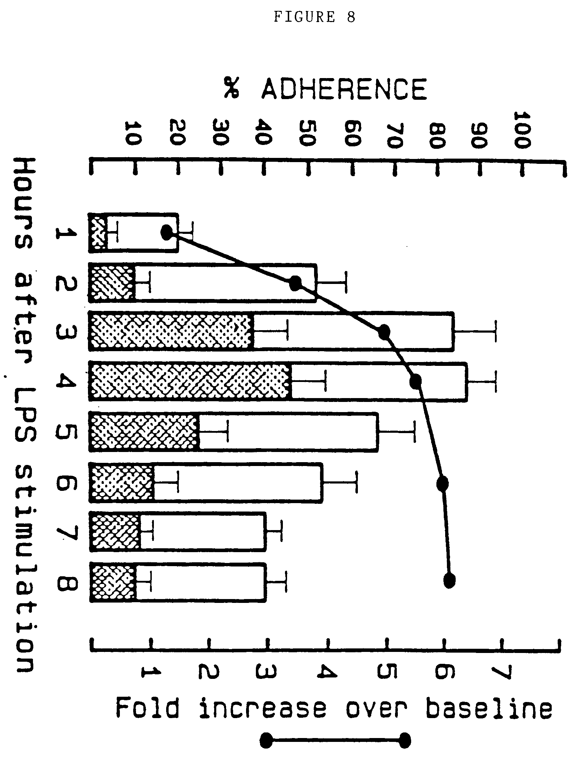

Figure 8 shows the changes over time in R6-5-D6 binding and adherence of PMNL to HUVEC monolayers. HUVEC monolayers were exposed to LPS (10 ng/ml) for the times indicated then washed by dipping 3 times in two changes of PBS. Adherence of PMNL preincubated (15 min, room temperature) in TS1/18 (1:200 dilution of ascites) or PBS was assessed using a visual assay at room temperature. R6-5-D6

EP 0 314 863 B1

binding was assessed using an EIA procedure (results shown from a representative experiment). Error bars, ± 1 s.d.; n, 4. Open bars plus the fine grid bars indicate the adherence of PMNL suspended in PBS; the fine grid bars indicate the adherence remaining after preincubation of PMNL in TS1/18. TS1/18 remained with the cells during the adherence assay.

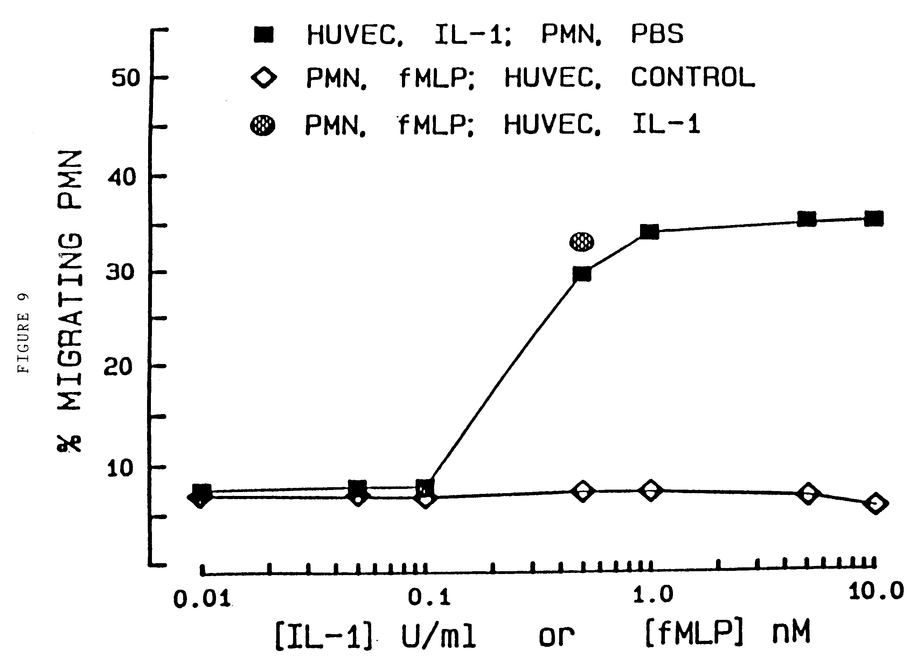

Figure 9 shows the effects of IL-1 and fMLP on the migration of PMNL through HUVEC monolayers. The effect of IL-1 was determined in the following manner: HUVEC monolayers were incubated for 4 hr, 37°C, in the presence of the indicated concentration of IL-1 and then washed by dipping 3 times in two changes of PBS. The percentage of cells migrating through the monolayer and between the endothelial cells and the substratum was determined at room temperature 1000 sec after allowing unstimulated PMNL to settle onto the monolayer. The effect of fMLP on migration through unstimulated HUVEC was determined after 15 min preincubation of the PMNL with the indicated concentration of fMLP at room temperature.

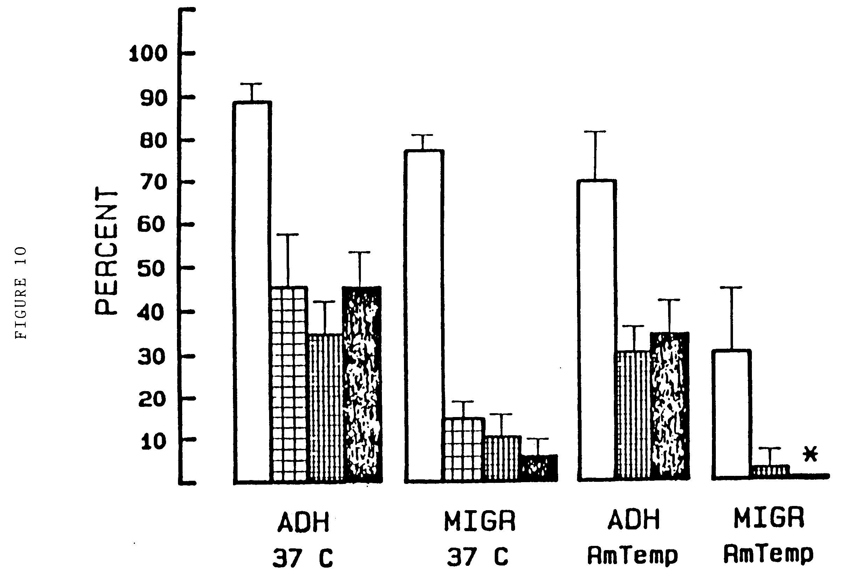

Figure 10 shows the inhibition of PMNL migration through LPS stimulated HUVEC by R6-5-D6 IgG and Fab preparations, and comparison with CD18-deficient PMNL. HUVEC incubated for 4 hr, 37°C, with 10 ng/ml LPS and 15 min with the monoclonal antibody or PBS prior to being washed and mounted in the adherence chamber. R6-5-D6 IgG and Fab at the concentration used in the preincubation (8 ug/ml and 48 ug/ml, respectively) were added to the leukocyte suspension prior to injection into the chamber. The entire adherence procedure was carried out at 37°C or room temperature (Rm Temp). p<0.01 (n=4) for each experimental condition compared to normal adult cells without monoclonal antibody. % Migration ± 1 s.d., of total cells originally coming in contact with the monolayer which migrate between the endothelial cells and substratum. % Adherence ± 1 s.d. Open bars, adult PMNL without monoclonal antibody;l course screen, Fab preparation; fine screen, IgG preparation; shaded bars, CD18-deficient PMNL. *, no migration seen.

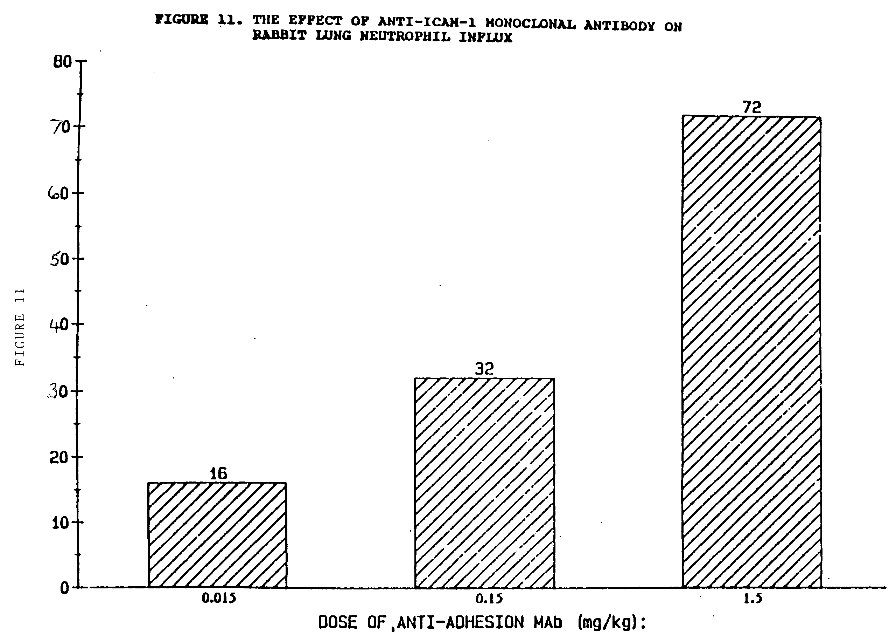

Figure 11 shows the effect of anti-ICAM antibody on the influx of neutrophils into inflamed rabbit lungs.

## DESCRIPTION OF THE PREFERRED EMBODIMENTS

Leukocyte cellular adhesion results from the interaction of cell surface glycoproteins of the CD18 complex. Such molecules comprise glycoproteins such as Mac-1, LFA-1, and p150,95. The key attribute of such glycoproteins is that these molecules play a role in cellular adhesion. Such glycoproteins are equivalently referred to herein as "members of the CD18 complex," or as "members of the CD18 family of molecules." The present invention derives from the discovery that ICAM-1 on endothelial cells binds to the members of the CD18 family of molecules on granulocytes responsible for mediating granulocyte-endothelial cell adhesion and that antagonists of ICAM-1 are capable of inhibiting such adhesion. Such inhibition provides a means for treating general, non-specific tissue inflammation.

The present invention derives in part from the discovery that granulocyte-endothelial cell adherence results from the interaction of glycoproteins of the CD18 family with ICAM-1. Since cellular adhesion is required in order that leukocytes may migrate to sites of inflammation and/or carry out various effector functions contributing to inflammation, agents which inhibit cellular adhesion will attenuate or prevent inflammation. A "non-specific defense system reaction" is a response mediated by leukocytes incapable of immunological memory. Such cells include granulocytes and macrophages. As used herein, inflammation is said to result from a response of the non-specific defense system, if the inflammation is caused by, mediated by, or associated with a reaction of the non-specific defense system. Examples of inflammation which result, at least in part, from a reaction of the non-specific defense system include inflammation associated with conditions such as: adult respiratory distress syndrome (ARDS) or multiple organ injury syndromes secondary to septicemia or trauma; reperfusion injury of myocardial or other tissues; acute glomerulonephritis; reactive arthritis; dermatoses with acute inflammatory components; acute purulent meningitis or other central nervous system inflammatory disorders; thermal injury; hemodialysis; leukapheresis; ulcerative colitis; Crohn's disease; necrotizing enterocolitis; granulocyte transfusion associated syndromes; and cytokine-induced toxicity.

The anti-inflammatory agents of the present invention are compounds capable of specifically antagonizing the interaction of the CD18 complex on granulocytes with ICAM-1 on endothelial cells. Such antagonists comprise: ICAM-1; a functional derivative of ICAM-1; a non-immunoglobulin antagonist of ICAM-1; an antibody capable of binding to ICAM-1; and a fragment of an antibody, the fragment being capable of binding to ICAM-1.

The natural binding ligand of the present invention is known as Intercellular Adhesion Molecule-1 ("ICAM-1"). ICAM-1 is a 76-97 Kd glycoprotein. ICAM-1 is not a heterodimer. The present invention is directed toward ICAM-1 and its "functional derivatives." A "functional derivative" of ICAM-1 is a compound which possesses a biological activity (either functional or structural) that is substantially similar to a biological activity of ICAM-1. Since ICAM-1, and its functional equivalents are capable of binding to

5

members of the CD18 complex, they may be employed as the anti-inflammatory agents of the present invention. The term "functional derivatives" is intended to include the "fragments," "variants," "analogs," or "chemical derivatives" of a molecule. A "fragment" of a molecule such as ICAM-1, is meant to refer to any polypeptide subset of the molecule. A "variant" of a molecule such as ICAM-1 is meant to refer to a molecule substantially similar in structure and function to either the entire molecule, or to a fragment thereof. A molecule is said to be "substantially similar" to another molecule if both molecules have substantially similar structures, or if both molecules possess a similar biological activity. Thus, provided that two molecules possess a similar activity; they are considered variants as that term is used herein even if one of the molecules contains additional amino acid residues not found in the other, or if the sequence of amino acid residues is not identical. An "analog" of a molecule such as ICAM-1 is meant to refer to a molecule substantially similar in function to either the entire molecule or to a fragment thereof. As used herein, a molecule is said to be a "chemical derivative" of another molecule when it contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed in Remington's Pharmaceutical Sciences (1980). "Toxin-derivatized" molecules constitute a special class of "chemical derivatives." A "toxin-derivatized" molecule is a molecule (such as ICAM-1 or an antibody) which contains a toxin moiety. The binding of such a molecule to a cell brings the toxin moiety into close proximity with the cell and thereby promotes cell death. Any suitable toxin moiety may be employed; however, it is preferable to employ toxins such as, for example, the ricin toxin, the diphtheria toxin, radioisotopic toxins, membrane-channel-forming toxins, etc. Procedures for coupling such moieties to a molecule are well known in the art.

Since granulocyte-endothelial cell adhesion requires both ICAM-1 and the CD18 complex, antibodies (or fragments thereof) which are capable of binding to ICAM-1 may be used as anti-inflammatory agents in accordance with the present invention. Since ICAM-1 is naturally expressed on the surfaces of some cells, the introduction of such cells into an appropriate animal, as by intraperitoneal injection, etc., will result in the production of antibodies capable of binding to ICAM-1. If desired, the serum of such an animal may be removed and used as a source of polyclonal antibodies capable of binding to ICAM-1. It is, however, preferable to remove splenocytes from such animals, to fuse such spleen cells with a myeloma cell line and to permit such fusion cells to form a hybridoma cell which secretes monoclonal antibodies capable of binding ICAM-1.

The hybridoma cells, obtained in the manner described above may be screened by a variety of methods to identify desired hybridoma cells that secrete antibody capable of binding to ICAM-1. In a preferred screening assay, such molecules are identified by their ability to inhibit the aggregation of Epstein-Barr virus-transformed cells. Antibodies capable of inhibiting such aggregation are then further screened to determine whether they inhibit such aggregation by binding to ICAM-1, or to a member of the CD18 complex. Any means capable of distinguishing ICAM-1 from members of the CD18 complex may be employed in such a screen. Thus, for example, the antigen bound by the antibody may be analyzed as by immunoprecipitation and polyacrylamide gel electrophoresis. If the bound antigen is a member of the CD18 complex then the immunoprecipitated antigen will be found to be a dimer, whereas if the bound antigen is ICAM-1 a single molecular weight species will have been immunoprecipitated. Alternatively, it is possible to distinguish between those antibodies which bind to members of the CD18 family of molecules from those which bind ICAM-1 by screening for the ability of the antibody to bind to cells such as granulocytes, which express LFA-1, but not ICAM-1. The ability of an antibody (known to inhibit cellular aggregation) to bind to granulocytes indicates that the antibody is capable of binding LFA-1. The absence of such binding is indicative of an antibody capable of recognizing ICAM-1. The ability of an antibody to bind to a cell such as a granulocyte may be detected by means commonly employed by those of ordinary skill. Such means include immunoassays, cellular agglutination, filter binding studies, antibody precipitation, etc.

The anti-aggregation antibodies of the present invention may alternatively be identified by measuring their ability to differentially bind to cells which express ICAM-1 (such as activated endothelial cells), and their inability to bind to cells which fail to express ICAM-1 (such as granulocytes). As will be readily appreciated by those of ordinary skill, the above assays may be modified, or performed in a different sequential order to provide a variety of potential screening assays, each of which is capable of identifying and discriminating between antibodies capable of binding to ICAM-1 versus members of the CD18 complex.

The anti-inflammatory agents of the present invention may be obtained by natural processes (such as, for example, by inducing an animal, plant, fungi, bacteria, etc., to produce a non-immunoglobulin antagonist of ICAM-1, or by inducing an animal to produce polyclonal antibodies capable of binding to ICAM-1); by synthetic methods (such as, for example, by using the Merrifield method for synthesizing polypeptides to synthesize ICAM-1, functional derivatives of ICAM-1, or protein antagonists of ICAM-1 (either immunoglobulin or non-immunoglobulin)); by hybridoma technology (such as, for example, to produce monoclonal antibodies capable of binding to ICAM-1); or by recombinant technology (such as, for example, to produce the anti-inflammatory agents of the present invention in diverse hosts (i.e., yeast, bacteria, fungi, cultured mammalian cells, etc.), or from recombinant plasmids or viral vectors). The choice of which method to employ will depend upon factors such as convenience, desired yield, etc. It is not necessary to employ only one of the above-described methods, processes, or technologies to produce a particular anti-inflammatory agent; the above-described processes, methods, and technologies may be combined in order to obtain a particular anti-inflammatory agent.

## A. Intercellular Adhesion Molecule-1 (ICAM-1)

The novel intercellular adhesion molecule ICAM-1 was first identified and partially characterized according to the procedure of Rothlein, R. et al. (J. Immunol. 137:1270-1274 (1986)), which reference is herein incorporated by reference. To detect the ICAM-1 molecule, monoclonal antibodies were prepared from spleen cells of mice immunized with cells from individuals genetically deficient in LFA-1 expression. Resultant antibodies were screened for their ability to inhibit the aggregation of LFA-1-expressing cells.

In detail, mice were immunized with EBV-transformed B cells from LAD patients which do not express the LFA-1 antigen. The spleen cells from these animals were subsequently removed, fused with myeloma cells, and allowed to become monoclonal antibody producing hybridoma cells. EBV-transformed B cells from normal individuals which express LFA-1 were then incubated in the presence of the monoclonal antibody of the hybridoma cell in order to identify any monoclonal antibody which was capable of inhibiting the phorbol ester mediated, LFA-1 dependent, spontaneous aggregation of the EBV-transformed B cells. Since the hybridoma cells were derived from cells which had never encountered the LFA-1 antigen no monoclonal antibody to LFA-1 was produced. Hence, any antibody found to inhibit aggregation must be capable of binding to an antigen that, although not LFA-1, participated in the LFA-1 adhesion process. Although any method of obtaining such monoclonal antibodies may be employed, it is preferable to obtain ICAM-1-binding monoclonal antibodies by immunizing BALB/C mice using the routes and schedules described by Rothlein, R. et al. (J. Immunol. 137:1270-1274 (1986)) with Epstein-Barr virus-transformed peripheral blood mononuclear cells from an LFA-1-deficient individuals. Such cells are disclosed by Springer, T.A., et al,. (J. Exper. Med. 160:1901-1918 (1984)).

In a preferred method for the generation and detection of antibodies capable of binding to ICAM-1, mice are immunized with either EBV-transformed B cells which express both ICAM-1 and LFA-1 or more preferably with TNF-activated endothelial cells which express ICAM-1 but not LFA-1. Such cells may be prepared according to the technique of Springer, T.A. et al., J. Exper. Med. 160:1901-1918 (1984), which reference is herein incorporated by reference. Although any such cell may be employed in the present invention, it is preferable to employ cells of the JY cell line (Terhost, C.T. et al., Proc. Natl. Acad. Sci. USA 73:910 (1976)). In a most preferred method for generating hybridoma cells which produce anti-ICAM-1 antibodies, a Balb/C mouse was sequentially immunized with the Epstein-Barr virus-transformed cell line, JY (Terhost, C.T., et al., Proc. Natl. Acad. Sci. (USA) 73:910 (1976)), and the differentiated cell line U937 (ATCC CRL-1593; American Type Culture Collection, Rockville, Md., U.S.A.). The cells may be cultivated in any suitable culture medium; however, it is most preferable to culture the cells in RPMI 1640 culture medium supplemented with 10% fetal calf serum and 50 ug/ml gentamicin (Gibco Laboratories, NY). The cells should be cultured under conditions suitable for mammalian cell proliferation (i.e., at a temperature of generally 37°C, in an atmosphere of 5% $CO_2$, at a relative humidity of 95%, etc.).

The spleen cells from immunized animals are removed, fused with myeloma cells and permitted to develop into antibody-producing hybridoma cells. The antibodies are screened for their ability to inhibit the LFA-1 dependent, phorbol ester induced aggregation of an EBV transformed cell line, such as JY cells, that expresses both the LFA-1 receptor and ICAM-1. As shown by Rothlein, R., et al. (J. Immunol. 137:1270-1274 (1986)), antibodies capable of inhibiting such aggregation are then tested for their ability to inhibit the phorbol ester induced aggregation of a cell line, such as SKW3 (Dustin, M. et al., J. Exper. Med. 165:672-692 (1987)) whose ability to spontaneously aggregate in the presence of a phorbol ester is inhibited by antibody capable of binding LFA-1 but is not inhibited by anti-ICAM-1 antibodies. Antibodies capable of inhibiting the phorbol ester induced aggregation of cells such as JY cells, but incapable of inhibiting the phorbol ester induced aggregation of cells such as SKW3 cells are probably anti-ICAM-1 antibodies. Alternatively, antibodies that are capable of binding to ICAM-1 may be identified by screening for antibodies which are capable of inhibiting the LFA-1 dependent aggregation of LFA-expressing cells (such as JY cells) but are incapable of binding to cells that express LFA-1 with little or no ICAM-1 (such as normal granulocytes) or are capable of binding to cells that express ICAM-1 but not LFA-1 (such as TNF-activated endothelial cells). Another alternative is to immunoprecipitate from cells expressing ICAM-1, LFA-1, or both, using antibodies that inhibit the LFA-1 dependent aggregation of cells, such as JY cells, and through SDS-PAGE or an equivalent method determine some molecular characteristic of the molecule precipitated with the antibody. If the characteristic is the same as that of ICAM-1 then the antibody can be assumed to be an anti-ICAM-1 antibody.

Using the above-described most preferred method for generating hybridoma cell lines which produce monoclonal antibodies capable of binding to ICAM-1, a hybridoma cell line was isolated which produced a preferred IgG2a anti-ICAM-1 antibody. This antibody was designated "R6•5•D6•E9•B2" (hereinafter referred to as "R6-5-D6"). The hybridoma cell line which produces antibody R6-5-D6 was designated "R6•5•D6•E9•B2." This cell line was deposited with the American Type Culture Collection (Rockville, Md., U.S.A.) on October 30, 1988, and given the designation HB 9580. The deposit was made under the terms of the Budapest Treaty.

## B. Uses of Assays of LFA-1 Dependent Aggregation

The above-described assays, capable of measuring CD18 complex dependent aggregation or adhesion, may be employed to identify agents which act as antagonists to inhibit the extent of cellular aggregation or adhesion. Such antagonists may act by impairing the ability of ICAM-1 to mediate aggregation or adhesion. Thus, such agents include immunoglobulins such as an antibody capable of binding to ICAM-1. Additionally, non-immunoglobulin agents (i.e., ICAM-1, functional derivatives of ICAM-1, or chemicals capable of binding to ICAM-1) may be examined, using the above-described assays, to determine whether they are antagonists of CD18-ICAM-1 interactions. Such agents can then be tested for their ability to inhibit granulocyte-endothelial cell adhesion using a suitable assay such as the following assay of granulocyte-endothelial cell adhesion.

Antagonists identified in this manner may be used for the same purposes as antibodies to ICAM-1. All such antagonists may be employed as the anti-inflammatory agent of the present invention.

## C. Assay of Granulocyte-Endothelial Cell Adhesion

The ability of polymorphonuclear leukocytes (PMNL) to adhere to human umbilical vein endothelial cells (HUVEC) provides a visual adherence assay which may be used to measure cellular adhesion in accordance with the present invention. Preferably, such an assay is conducted using the method of Smith, C.W., et al. (J. Clin. Invest. 63:221-229 (1979)), with the exception that adherence to HUVEC monolayers is assessed instead of adherence to protein-coated glass. In a preferred assay, HUVEC monolayers, preferably on 25 mm round glass cover slips, are prepared and immediately inserted into adherence chambers specifically made for use with an inverted microscope and phase contrast optics. Such chambers preferably consist of two metal plates designed to hold two 25 mm round cover glasses separated by a Sykes-Moore chamber O-ring (Bellco). PMNL are introduced into the chamber so as to permit contact with HUVEC monolayer. The number of PMNL in contact with the monolayer may be determined by microscopic examination. The percentage of cells remaining in contact with the monolayer after washing provides a measure of cellular adherence. Additionally, it is possible to monitor the migration of adherent PMNL through the HUVEC monolayer.

In order to determine whether an agent is a potential anti-inflammatory compound, HUVEC and/or PMNL may be pretreated with the agent, preferably for between 1-60 minutes, and the level of cellular adherence measured. Agents capable of inhibiting inflammation are identified by their ability to block cellular adhesion in such an assay.

## D. Uses of Intercellular Adhesion Molecule-1 (ICAM-1)

ICAM-1 is a binding partner of the members of the CD18 complex. As such, ICAM-1 or its functional derivatives can be used to prevent endothelial cells from adhering to granulocytes by binding to the CD18 molecule on the surface of a granulocyte, and thus preventing the CD18 molecules of the granulocyte from binding to the ICAM-1 of endothelial cells.

ICAM-1, or its functional derivatives are highly suitable as anti-inflammatory agents in a mammalian subject. Significantly, such agents differ from conventional anti-inflammatory agents in that they do not have other side effects such as nephrotoxicity which are found with conventional agents.

### E. Uses of Antagonists of ICAM-1

Antibodies (especially monoclonal antibodies) or non-immunoglobulin antagonists of ICAM-1 can be used to prevent granulocytes from adhering to endothelial cells by binding to the ICAM-1 of endothelial cells, and thus preventing the ICAM-1 of the endothelial cells from binding to the CD18 molecules on the surface of the granulocyte. Mac-1 and LFA-1 are capable of binding to ICAM-1. Thus, these molecules are examples of non-immunoglobulin antagonists of ICAM-1. Through the use of the above-described assays, additional non-immunoglobulin antagonists of ICAM-1 dependent granulocyte adhesion can be identified. Since granulocytes are implicated in mediating tissue damage in inflammatory conditions mediated by the non-specific defense system (such as adult respiratory distress syndrome, etc.) and since granulocytes must adhere to endothelial cells in order to migrate to inflammatory sites, the blocking of granulocyte-endothelial cell adhesion by an anti-ICAM-1 antibody or by a non-immunological antagonist of ICAM-1 will prevent granulocyte-induced tissue damage.

Antibodies (especially monoclonal antibodies), or non-immunoglobulin antagonists of ICAM-1 capable of binding to ICAM-1 are highly suitable as anti-inflammatory agents in a mammalian subject. Significantly, such agents differ from conventional anti-inflammatory agents in that they do not have other side effects such as nephrotoxicity which are found with conventional agents.

### F. Administration of the Anti-Inflammatory Agents of the Present Invention

The anti-inflammatory effects of ICAM-1 may be obtained by providing to a patient the entire ICAM-1 molecule, or any therapeutically active peptide fragments thereof.

ICAM-1 and its functional derivatives may be obtained either synthetically, through the use of recombinant DNA technology, or by proteolysis. The therapeutic advantages of ICAM-1 may be augmented through the use of functional derivatives of ICAM-1 molecules possessing additional amino acid residues added to enhance coupling to carrier or to enhance the activity of the ICAM-1. The scope of the present invention is further intended to include functional derivatives of ICAM-1 which lack certain amino acid residues, or which contain altered amino acid residues, so long as such derivatives exhibit the capacity to effect cellular adhesion.

Both the antibodies of the present invention and the ICAM-1 molecule disclosed herein are said to be "substantially free of natural contaminants" if preparations which contain them are substantially free of materials with which these products are normally and naturally found.

The present invention extends to antibodies, and biologically active fragments thereof, (whether polyclonal or monoclonal) which are capable of binding to ICAM-1. Such antibodies may be produced either by an animal, or by tissue culture, or by recombinant DNA means.

In providing a patient with antibodies, or fragments thereof, capable of binding to ICAM-1, or when providing ICAM-1 (or a functional derivative thereof) to a recipient patient, the dosage of administered agent will vary depending upon such factors as the patient's age, weight, height, sex, general medical condition, previous medical history, etc. In general, it is desirable to provide the recipient with a dosage of antibody which is in the range of from about 1 pg/kg to 10 mg/kg (amount of agent/body weight of patient), although a lower or higher dosage may be administered. When providing ICAM-1 molecules or their functional derivatives to a patient, it is preferable to administer such molecules in a dosage which also ranges from about 1 pg/kg to 10 mg/kg (amount of agent/body weight of patient) although a lower or higher dosage may also be administered.

The anti-inflammatory agents of the present inventon are intended to be provided to recipient subjects in an amount sufficient to suppress inflammation. An amount is said to be sufficient to "suppress" inflammation if the dosage, route of administration, etc. of the agent are sufficient to attenuate or prevent inflammation.

The anti-inflammatory agents of the present invention may be provided either prior to the onset of inflammation (so as to suppress an anticipated inflammation) or after the initiation of inflammation.

Both the desired antibody and ICAM-1 itself may be administered to patients intravenously, intramuscularly, subcutaneously, enterally, or parenterally. When administering antibody or ICAM-1 by injection, the administration may be by continuous infusion, or by single or multiple boluses.

A composition is said to be "pharmacologically acceptable" if its administration can be tolerated by a recipient patient. Such an agent is said to be administered in a "therapeutically effective amount" if the amount administered is physiologically significant. An agent is physiologically significant if its presence results in a detectable change in the physiology of a recipient patient.

The antibody and ICAM-1 molecules of the present invention can be formulated according to known methods to prepare pharmaceutically useful compositions, whereby these materials, or their functional derivatives, are combined in an mixture with a pharmaceutically acceptable carrier vehicle. Suitable vehicles and their formulation, inclusive of other human proteins, e.g., human serum albumin, are described, for example, in Remington's Pharmaceutical Sciences (16th ed., Osol, A., Ed., Mack, Easton PA (1980)). In order to form a pharmaceutically acceptable composition suitable for effective administration, such compositions will contain an effective amount of antibody or ICAM-1 molecule, or their functional derivatives, together with a suitable amount of carrier vehicle.

Additional pharmaceutical methods may be employed to control the duration of action. Control release preparations may be achieved through the use of polymers to complex or absorb antibody or ICAM-1, or their functional derivatives. The controlled delivery may be exercised by selecting appropriate macromolecules (for example polyesters, polyamino acids, polyvinyl, pyrrolidone, ethylenevinylacetate, methylcellulose, carboxymethylcellulose, or protamine, sulfate) and the concentration of macromolecules as well as the methods of incorporation in order to control release. Another possible method to control the duration of action by controlled release preparations is to incorporate antibody or ICAM-1 molecules, or their functional derivatives, into particles of a polymeric material such as polyesters, polyamino acids, hydrogels, poly(lactic acid) or ethylene vinylacetate copolymers. Alternatively, instead of incorporating these agents into polymeric particles, it is possible to entrap these materials in microcapsules prepared, for example, by coacervation techniques or by interfacial polymerisation, for example, hydroxymethylcellulose or gelatine-microcapsules and poly(methylmethacylate) microcapsules, respectively, or in colloidal drug delivery systems, for example, liposomes, albumin microspheres, microemulsions, nanoparticles, and nanocapsules or in macroemulsions. Such techniques are disclosed in Remington's Pharmaceutical Sciences (1980).

Having now generally described the invention, the same will be more readily understood through reference to the following examples which are provided by way of illustration, and are not intended to be limiting of the present invention, unless specified. The results presented in the following examples are presented as means ± 1 standard deviation, and N = the number of separate experiments. Statistical assessments were made using analysis of variance and Dunnett's t Test, or Student's t Test.

## EXAMPLE 1

**Adherence of PMNL to HUVEC**

Isolation of Polymorohonuclear Leukocytes (PMNL). PMNL obtained from healthy adult individuals and two patients with a severe form of CD18 deficiency (Anderson, D.C., et al., J. Infect. Dis. 152:668-689 (1985)) were purified from citrate anticoagulated, dextran-sedimented venous blood samples over Ficoll-Hypaque gradients and were suspended in Dulbecco's phosphate-buffered saline (PBS; GIBCO, Grand Island, NY), pH 7.4, containing 0.2% dextrose as described (Anderson, D.C., et al., J. Infect. Dis. 143:447-459 (1981)). PMNL were maintained at 4°C in PBS for up to 4 hours at a concentration of $10^7$/ml.

Monoclonal Antibodies. Monoclonal antibodies directed against the CD18 complex used in these studies included dilutions of ascites fluid or culture supernatant, and preparations of IgG and F(ab) fragments.

Preparation of Human Umbilical Vein Endothelial Cells (HUVEC). HUVEC were harvested (Gimbrone, M.A., Prog. Hemostas. Thromb. 3:1-28 (1976)) and characterized as to acetylated low density lipoprotein (acLDL) binding (Voyta, J.C., et al., J. Cell. Biol. 99:2034-2040 (1984)) and factor VIII expression (Jaffe, E.A., et al., J. Clin. Invest. 52:2745-2757 (1973)). Cells from 5-10 umbilical cords were pooled and plated in RPMI 1640 containing 20% Fetal Calf Serum (FCS), antibiotics, heparin (0.1 mg/ml), and endothelial cell growth factor (0.05 mg/ml), and maintained for 3-4 days at 37°C, 5% $CO_2$ humidified atmosphere. Visually confluent mono-layers on gelatin (0.1%) and fibronectin (5 ug/cm$^2$) coated 25 mm round blass coverslips were prepared from first passage cells harvested with 0.05% trypsin and 0.02% EDTA in PBS. Monolayers were prepared in fibronectin (5 ug/cm$^2$) coated 96 well microtiter plates from first and second passage cells and grown to confluence (1-3 days). HUVEC were pretreated with various concentrations of LPS (Sigma, E. coli 026:B6), or IL-1 (Genzyme, cell derived) for varying lengths of time.

Adherence Assay. A visual adherence assay was utilized as described by Smith, C.W. (J. Clin. Invest. 63:231-229 (1979)) with the exception that adherence to HUVEC monolayers wasassed instead of adherence to protein-coated glass. HUVEC monolayers on 25 mm round blass coverslips were washed by dipping 3 times in two changes of PBS and immediately inserted into the adherence chambers specifically made for use with an inverted microscope and phase contrast optics. The chambers consisted of two metal plates designed to hold two 25 mm round cover glasses separated by a Sykes-Moore chamber o-ring (Bellco). Within the closed compartment PMNL could be observed as they contacted the HUVEC monolayer. PMNL suspended in PBS at a concentration of $10^6$ cells/ml were injected into the chamber and allowed to settle onto the monolayer for a period of 500 seconds. The number of PMNL in contact with the monolayer was determined by counting at least 10 microscopic fields (50X objective), and the chamber inverted for an additional 500 sec. The percentage of cells remaining in contact with the monolayer was determined and is expressed in the Results as percent adherence. The percentage of cells migrating through the monolayer (a subset of the adherent cells) was also determined. In blocking experiments, HUVEC and/or PMNL were pretreated with monoclonal antibodies for 15 min and then washed prior to evaluating adherence. In some experiments monoclonal antibodies were retained with the cells during the adherence assay.

Assessment of Monoclonal Antibody Binding to Cells. Immunofluorescence flow cytometry was performed as described by Anderson, D.C., et al. (J. Clin. Invest. 74:536-551 (1984)) using monoclonal antibodies and fluorescein isothiocyanate (FITC)-conjugated antibody to mouse IgG. Surface-stained cells were fixed in 1% paraformaldehyde and analyzed in a flow cytometer (Ortho, Cytofluorograph). Background fluorescence was determined after incubation with either non-immune ascites or X63 IgG1 control antibody.

Fluorescence microscopy was performed on paraformaldehyde (1%, 15 min, room temperature) fixed cells washed in PBS and incubated 30 min in PBS containing 2% human serum albumin (HSA) and 1% glycine. The binding of monoclonal antibodies was detected by use of a second antibody to mouse IgG conjugated with FITC or rhodamine isothiocyanate (RITC). Cells were examined using a Leitz Diaplan fluorescent microscope. Background fluorescence was determined after incubation of cells with either non-immune ascites or X63 IgG1 control antibody.

An enzyme immunoassay (EIA) was used to assess binding of monoclonal antibodies to cell monolayers. Confluent HUVEC grown in fibronectin-coated (5 ug/mm$^2$) 96 well plates were fixed by addition of 1% paraformaldehyde in PBS for 15 min at room temperature. The wells were washed 3 times with PBS and incubated in 2% bovine serum albumin (BSA) for 30 min. After removal of BSA, monoclonal antibody was added and incubated at 37°C for 1 hour, washed three times in PBS and then incubated for 1 hour in goat-antimouse IgG, IgM, IgA (Zymed) conjugated with alkaline phosphatase (1:500 dilution). After washing, substrate (p-nitrophenyl disodium phosphate, 1 mg/ml, in buffer, pH 9.8) was added and incubated for 30 min at room temperature. Plates were read at 405 nm in a Titertek (Flow) reader.

## EXAMPLE 2

### Effect of fMLP on Adhesion

fMLP (f-Met-Leu-Phe), at concentrations of between 0.5-2.0 mM has been found to reduce cellular adhesion (Charo, I.F., et al., J. Immunol. 136:3412-3419 (1986); Tonnesen, M.G., et al., J. Clin. Invest. 74:1581-1592 (1984)).

Chemotactic factors (CF) have been shown to be capable of increasing or decreasing PMNL adherence to protein-coated glass or plastic (Anderson, D.C., et al., J. Infect. Dis. 143:447-459 (1981); Smith, C.W., et al., J. Clin. Invest. 65:804-812 (1980); Anderson, D.C., et al., J. Lab. Clin. Med. 101:881-895 (1983); Anderson, D.C., et al., J. Clin. Invest. 68:863-874 (1981)). The observed effect of the CF is dependent on the concentration of the CF and on prior exposure of leukocytes to CF. Shape change inducing (Haslett, C., et al., Amer. J. Pathol. 119:101-110 (1985)) concentrations of fMLP (0.5-2.0 nM) will decrease adherence to protein-coated glass if cells have been exposed previously to a lower concentration of the chemotactic factor. Such "priming" can occur at concentrations as low as 0.01 nM fMLP (Smith, C.W., et al., J. Clin. Invest. 65:804-812 (1980)) and leads to a redistribution of the cell's "adhesion sites" upon subsequent chemotactic exposure. Great care must be taken to avoid activation or priming during isolation. (Haslett, C., et al., Amer. J. Pathol. 119:101-110 (1985)). In addition to the physiologic response of the PMNL to chemotactic stimulation, another factor potentially influencing the level of adherence is found in the adherence assay. The procedure used in the present invention does not utilize shear stress. Rather it simply assesses attachment. While shear stress is obviously important in vivo (Schmid-Schoenbein, G.W., et al., Circ. Res. 36:173-181 (1975); Schmid-Schoenbein, G.W., et al., Microvas. Res. 19:45-58 (1980)), its inclusion in an adherence assay in vivo must be carefully controlled (Forrester, J.V., et al., J. Cell. Sci. 70:93-105 (1984)), a circumstance not possible with simple washing procedures. Thus, in the present assays, the CD18 glycoprotein complex was assessed for its importance in attachment of PMNL to HUVEC, and no studies were performed to assess the role of the CD18 complex in resistance of attached PMNL to carefully controlled shear stress (Gallik, S., et al., Fed. Proc. 46:1043a (1987)).

In contrast to the findings of Charo, I.F., et al. (J. Immunol. 136:3412-3419 (1986)) and Tonneson, M.G., et al. (J. Clin. Invest. 74:1581-1592 (1984)), one aspect of the present invention is the discovery that very low concentrations of fMLP are capable of increasing the adherence of neutrophils to HUVEC.

### EXAMPLE 3

**Time and Dose Dependent Augmentation of Adherence by Inflammatory Mediators**

The baseline adherence of PMNL to HUVEC varied with each preparation of HUVEC from a low of 3.5 ± 1.0% to a high of 35.5 ± 1.0% of the PMNL adhering to the mono-layer. The mean ± 1 s.d. for 24 preparations of HUVEC was 16.9 ± 7.5%. Adherence was significantly increased following stimulation of the PMNL by fMLP (Figure 1). Statistically significant increases were obtained at 0.1 nM fMLP (Figure 1) and maximal adherence (62.5 ± 5.3%; n, 8; 10 nM fMLP) was approached at a concentration of 0.5 nM (53.8 ± 9.8%; n, 23). IL-1 stimulation of the HUVEC significantly increased adherence of PMNL at 0.05 U/ml with near maximal adherence at a concentration of 0.5 U/ml (Figure 1; 81.2 ± 7.4%; n, 21). Near maximal adherence was attained at 10 ng/ml LPS (80.1 ± 7.6%; n, 25). Each of the latter two stimuli promoted 100% adherence after a 4 hour incubation with the HUVEC provided the concentrations exceeded 2 U/ml IL-1 or 30 ng/ml LPS The kinetics of the effects of IL-1 and LPS on HUVEC mediated adherence were very similar (Figure 2), each peaking at 3.5-4 hours and diminishing by approximately 50% at seven hours.

### EXAMPLE 4

**Binding of Monoclonal Antibodies to PMNL and HUVEC --Effects of fMLP, IL-1 and LPS Stimulation**

TS1/18 (anti-common (beta) subunit of the CD18 molecule), TS1/22 (anti-LFA-1 alpha subunit) (Sanchez-Madrid, F., et al., J. Exper. Med. 158:586-602 (1983)), OKM1 (anti-Mac-1 alpha subunit; Ortho Diagnostics), and W6/32 (anti-HLA framework antibody, produced by American Type Culture Collection strain HB95, ATCC, Rockville, Md., U.S.A.) all bound to peripheral mononuclear lymphocytes (PMNL). Flow cytometry, using an anti-mouse antibody labeled with fluorescein isothiocyanate (FITC), revealed that the monoclonal antibodies reactive with the αMac-1 and β subunits exhibited 3-5 fold enhanced binding following stimulation of the PMNL with 10 nM f-met-leu-phe (fMLP). Binding of monoclonal antibodies reactive with the αLFA-1 subunit and the class I MHC antigen did not increase following chemotactic stimulation (Anderson, D.C., et al., J. Immunol. 137:15-27 (1986)). R6-5-D6 and W6/32 bound to HUVEC. Enzyme-immunoassay (EIA) (using an alkaline phosphatase-labeled anti-mouse antibody) revealed that exposure of HUVEC to 20 ng/ml LPS resulted in a 4-5 fold increase at 4 hours and 5-7 fold increase at 7 hours in binding of R6-5-D6. W6/32 binding was not increased at 4 hours and increased by less than 20% following a 24 hour stimulus with LPS.

Immunofluorescence (IF) microscopy revealed a uniform distribution of R6-5-D6 over the surface of each endothelial cell following paraformaldehyde fixation. In the unstimulated monolayer, all cells exhibited fluorescence with about 20% obviously staining brighter than the average cell. After a 4 hour stimulation with IL-1 (0.5 U/ml) or LPS (10 ng/ml) the overall intensity of the staining was increased on all cells and about 50% were distinctly bright. Under maximum resolution (1200X magnification) the fluorescence was seen to be punctate with a uniform spacing of less than 0.5 um. IF microscopy of monolayers with attached PMNL revealed no staining of PMNL and an unstained area on the surface of the endothelial cell occupied by the neutrophil. The lack of binding to PMNL was confirmed by flow cytometry in that the fluorescence intensity was not above the non-immune ascites control. In marked contrast, the distribution of TS1/18 was confined to the attached PMNL with no staining of the HUVEC.

### EXAMPLE 5

**Effects of Monoclonal Antibodies on Adherence -- Pretreatment of PMNL**

Preincubation of the PMNL with the various monoclonal antibodies revealed a profound effect of TS1/18. Maximal inhibition of adherence by TS1/18 was attained at a 1:800 dilution of ascites fluid and at 5 ug/ml of the IgG1 preparation. As shown in Figure 3, this monoclonal antibody significantly reduced baseline adherence and completely blocked the enhancement caused by fMLP stimulation of the PMNL. In contrast, OKM1 was partially effective in blocking the fMLP enhanced adherence, and both TS1/22 and R6-5-D6 were without effect on either the baseline or the fMLP enhanced adherence. Experiments employing LPS and IL-1 stimulated HUVEC (Figure 4) revealed that pretreatment of the PMNL with the various monoclonal antibodies yielded quantitatively different results. TS1/18 inhibited the LPS and IL-1 enhanced adherence by about 50%. As with unstimulated HUVEC, TS1/18 pretreatment of PMNL completely blocked fMLP augmented PMNL adherence to LPS stimulated experiments, combinations of each monoclonal antibody with TS1/18 did not further reduce the adherence below that caused by TS1/18 alone. Increasing the concentration of TS1/18 either as ascites fluid or as isolated IgG1 did not reduce further the LPS stimulated adherence.

## EXAMPLE 6

**Effects of Monoclonal Antibodies on Adherence -- Pretreatment of HUVEC**

Preincubation of the HUVEC with the various monoclonal antibodies gave qualitatively different results. As shown in Figure 5, R6-5-D6 was the only monoclonal antibody effective in reducing the enhancement caused by IL-1 or LPS stimulation. In the experiments shown in this figure, the inhibition was not complete, averaging 73%. This effect of R6-5-D6 was maximal at a 1:800 dilution of the ascites fluid and at 4 ug/ml IgG2a fraction of ascites (percent inhibition at 12 ug/ml, 59.2, n, 3; at 4 ug/ml, 60.1, n, 6; and at 2 ug/ml, 38.3, n, 3). F(ab) fragments were maximally active at 48 ug/ml (percent inhibition at 48 ug/ml, 56.6, n, 3; at 24 ug/ml, 34.0, n, 3). When fMLP activated PMNL were used (Figure 6), R6-5-D6 was as effective as TS1/18 in blocking the adherence increment caused by chemotactic stimulation. Furthermore, it blocked fMLP augmentation of PMNL adherence to IL-1 and LPS stimulated HUVEC.

## EXAMPLE 7

**Effects of Monoclonal Antibodies on Adherence to Paraformaldehyde-Fixed HUVEC**

The above experiments involved exposure of viable HUVEC monolayers to the monoclonal antibodies for 15 minutes at 37°C. Thus, one explanation of the results of the above-described experiments is that R6-5-D6 was inducing a response in the endothelial cells which changed their adhesive properties. Such a conclusion is refuted by the observation (Table 1) that R6-5-D6 was able to inhibit the adherence of PMNL to paraformaldehyde-fixed HUVEC monolayers to the same degree as it inhibited the adherence of PMNL to viable HUVEC mono-layers. There was high adherence of PMNL to LPS stimulated endothelial cells, and fMLP stimulation of the PMNL augmented this adherence. R6-5-D6 significantly reduced adherence.

TABLE 1

| Inhibition of PMNL Adherence to Paraformaldehyde-Fixed HUVEC by Monoconal Antibody R6-5-D6 | | | |
|---|---|---|---|
| Pretreatment of | | n | % Adherence |
| PMNL | HUVEC | | |
| PBS | LPS | 12 | 61.8 ± 5.0 |
| PBS | LPS, R6-5-D6 | 9 | 20.0 ± 4.5** |
| PBS | LPS, W6/32 | 2 | 54.5 |
| fMLP | LPS | 10 | 87.3 ± 11.3 |
| fMLP | LPS, R6-5-D6 | 10 | 26.7 ± 16.3 |
| fMLP | LPS, W6/32 | 2 | 71.0 |
| PBS | Control | 4 | 18.5 ± 1.8 |
| fMLP | Control | 4 | 50.0 ± 4.9 |
| fMLP | Control, R6-5-D6 | 4 | 8.0 ± 1. 2** |

HUVEC were incubated without (control) or with 10 ug/ml LPS for 4 hr at 37 ° C, washed in PBS and then fixed for 15 min at room temperature in 1% paraformaldehyde. The monolayers were again washed in PBS and then incubated for 15 min in PBS containing 1% HSA (Human Serum Albumin) and 1% glycine. Monolayers were again washed and incubated for 30 min with PBS. R6-5-D6 (1:400 dilution of ascites) or W6/32 (1:200 dilution of ascites) following which adherence of control of fMLP (0.5 nM) activated PMNL was determined. *, p<0.01; , p<0.001, compared to condition without monoclonal antibody. % Adherence ± 1 s.d. The number of separate tests is shown by "n".

## EXAMPLE 8

### Direct Comparison of Inhibitory Effects of TS1/18 and R6-5-D6

Since R6-5-D6 gave results qualitatively similar to TS1/18 in that it completely blocked the enhancement caused by fMLP stimulation of the PMNL and partially blocked the enhancement caused by LPS or IL-1 stimulation of the HUVEC, direct comparison of these two monoclonal antibodies on the same preparations of PMNL and HUVEC was undertaken. Table 2 shows that the two monoclonal antibodies gave almost identical results. Furthermore, in experiments where the PMNL were blocked with TS1/18 and the HUVEC were blocked with R6-5-D6, inhibition of LPS enhanced adherence was no greater than with either monoclonal antibody alone. This comparison was further extended by the use of PMNL from a patient deficient in the CD18 complex. Neither TS1/18 nor R6-5-D6 exhibited any inhibitory effect n the IL-1 enhanced adherence of the deficient PMNL to normal HUVEC (Figure 7). The same lack of effect was seen on adherence of CD18 deficient PMNL to LPS stimulated HUVEC. A CD18-independent component to the LPS and IL-1 enhancement is clearly shown by the monoclonal antibodies and CD18-deficient leukocytes.

TABLE 2

| Comparison of the Inhibitory Activity of Monoclonal Antibodies R6-5-D6 and TS1/18 for PMNL Adherence to HUVEC | | | |
|---|---|---|---|
| Pretreatment of | | n | % Adherence |
| PMNL | HUVEC | | |
| PBS | Control | 5 | 15.0 ± 3.7 |
| PBS | Control, R6-5-D6 | 4 | 7.0 ± 1.4* |
| TS1/18 | Control | 4 | 6.3 ± 2.2* |
| PBS | IL-1 | 9 | 79.4 ± 9.6 |
| PBS | IL-1, R6-5-D6 | 13 | 33.0 ± 5.5* |
| TS1/18 | IL-1 | 8 | 30.4 ± 5.3* |
| TS1/18 | IL-1, R6-5-D6 | 4 | 31.3 ± 6.2* |

HUVEC were incubated without (control) and with IL-1 (0.5 U/ml) for 4 hr at 37°C, then exposed to R6-5-D6 (1:400 dilution of ascites) or PBS for 15 min. The monolayers were then washed and the adherence of PMNL preincubated in either PBS or TS1/18 (1:400 dilution of ascites) was assessed. *, p<0.01, compared to condition without monoclonal antibody. The number of separate tests is shown by "n".

EP 0 314 863 B1

**EXAMPLE 9**

**Kinetics of CD18-Dependent and CD18-Independent Adherence**

The time course of LPS and IL-1 effects on R6-5-D6 binding to HUVEC was evaluated. The amount of R6-5-D6 bound to HUVEC increased markedly over the first 3 hours and remained high over the 8 hours of observation as revealed by the EIA procedure (Figure 8). This was in contrast to the changes seen in adherence of unstimulated PMNL over the same time period (Figure 2). The diminishing adherence seen after four hours stimulation of HUVEC with IL-1 or LPS apparently could not be accounted for by reductions in the amount of the endothelial surface factor recognized by R6-5-D6. Therefore, the contribution of the CD18 independent determinants of PMNL adherence was evaluated. Adherence of PMNL pretreated with saturating concentrations of TS1/18 to LPS (Figure 8) or IL-1 stimulated HUVEC peaked at four hours, and fell by 70% at 6 hr and by 79.8% at 8 hr. The CD18 contribution to adherence over this time, as reflected by the difference in the adherence of TS1/18 treated and untreated PMNL, fell by 9.5% at 6 hr and by 26.0% at 8 hours. Thus, the largest contribution to the diminishing adherences after 4 hours appeared to be the CD18-independent factor(s). CD18-deficient PMNL and R6-5-D6 pretreatment of stimulated HUVEC revealed adherence changes over time comparable to those seen with TS1/18 pretreatment of PMNL (Table 3).

17

TABLE 3

| Time Dependent Changes in PMNL Adherence to LPS Treated HUVEC Monolayers and the Inhibitory Activity of Monoclonal Antibodies TS1/18 and R6-5-D6 | | | |
|---|---|---|---|
| Pretreatment of | | n | % Adherence |
| PMNL | HUVEC | | |
| PBS | LPS, 4 hr | 5 | 88.2 ± 7.0 |
| TS1/18 | LPS, 4 hr | 5 | 46.2 ± 4.9 |
| PBS | LPS, 4 hr, R6-5-D6 | 4 | 46.8 ± 5.9 |
| CD18-Def. | LPS, 4 hr | 4 | 50.5 ± 6.4 |
| PBS | LPS, 6 hr | 5 | 52.0 ± 10.7 |
| TS1/18 | LPS, 6 hr | 9 | 13.7 ± 3.8 |
| PBS | LPS, 6 hr, R6-5-D6 | 4 | 23.7 ± 4.7 |
| CD18-Def. | LPS, 6 hr | 4 | 13.0 ± 1.2 |
| PBS | LPS, 8 hr | 4 | 40.4 ± 3.1 |
| TS1/18 | LPS, 8 hr | 4 | 9.3 ± 2.6 |
| PBS | LPS, 8 hr, R6-5-D6 | 4 | 10.1 ± 3.3 |
| CD18-Def. | LPS, 8 hr | 4 | 11.3 ± 2.6 |

HUVEC were incubated with LPS (10 ng/ml) for time indicated, washed by dipping 3 times in two changes of PBS and then exposed to PBS or R6-5-D6 IgG (12 ug/ml) for 15 min. The HUVEC monolayers were washed again. Adherence of adult PMNL suspended in PBS or TS1/18 (1:400 dilution of ascites), or CD18-deficient (CD18-Def.) PMNL was determined using a visual assay at room temperature. % Adherence ± 1.s.d. The number of separate tests is shown by "n".

EP 0 314 863 B1

## EXAMPLE 10

**Requirements for PMNL Migration through HUVEC Monolayers**

A portion of the PMNL that adhere to the HUVEC has been observed to move through the monolayer and migrate between the HUVEC and the substratum. Tonnesen, M.G., et al., J. Clin. Invest. 74:1581-1592 (1984); Smedly, L.A., et al., Fed. Proc. 42:386a (1983); Gimbrone, M.A., et al., J. Clin. Invest. 74:1552-1555 (1984); Beesley, J.E., et al., J. Cell. Sci. 38:237-248 (1979); Armstrong, P.S., et al., J. Cell. Biol. 65:439-462 (1975)). These neutrophils assumed the bipolar configuration typical of motile PMNL though they appeared greatly flattened within this space. The percent of PMNL exhibiting this behavior was very low both under baseline conditions (<1%; n, 45) and when PMNL were exposed to fMLP (Figure 9). However, when the HUVEC were stimulated with IL-1 or LPS, the percent migrating through the monolayer increased significantly (33.3 ± 10.3%; n, 25; 10 ng/ml LPS, Figure 9). When the adherence assay was performed at 37°C, this behavior on LPS stimulated monolayers was exhibited by a significantly greater percentage of the cells (78.4 ± 4.2%; n, 8; $p<0.01$), though the percent adherence was increased somewhat less by this increase in temperature (Figure 10). R6-5-D6 and TS1/18 were both equally effective in blocking the migration of PMNL through the HUVEC monolayer. With the adherence assay performed at room temperature, the percentage of cells migrating through IL-1 stimulated (0.5 U/ml) monolayers was 30.6 ± 12.6% (n, 17) compared to 1.6 ± 1.6% (n, 17; $p<0.01$) or 5.0 ± 1.2% (n, 3; $p<0.05$) when PMNL were preincubated in TS1/18 (1:400 dilution of ascites) or OKM1 (1:200 dilution of ascites), respectively, or 2.5 ± 1.7% (n, 6; $p<0.01$) when the HUVEC monolayer was preincubated with R6-5-D6 (1:400 dilution of ascites). TS1/22 and W6/32 were without inhibitory effect (% migrating cells, 36.0 ± 11.5%; n, 3; and 32.2 ± 7.9%, n, 3, respectively). R6-5-D6 IgG and F(ab) preparations were evaluated at 37°C. Both produced significant inhibition of migration and adherence, both at 37°C and at room temperature. The percent inhibition of adherence at 37°C by R6-5-D6 F(ab) and IgG preparations was 51 and 60, respectively, while the percent inhibition of migration was 81 and 86. At room temperature, percent inhibition of adherence by R6-5-D6 IgG was 59, and percent inhibition of migration was 92.

The importance of the CD18 complex in transendothelial migration in vitro was further demonstrated by the results with CD18-deficient cells. As shown in Figure 10, they did not migrate at room temperature and migrated very little at 37°C.

## EXAMPLE 11

**Intracellular Location of Mac-1 and P150,95**

In normal unstimulated PMNL and monocytes, Mac-1 and p150,95 are present in an intracellular vesicular compartment as well as on the cell surface (Jones, D.H., et al., Pedi. Res. 21:312a (1987); Miller, L.J., et al., J. Clin. Invest. 80:185-191 (1987)). Exposure to chemotactic stimuli (for example, C5a and f-Met-Leu-Phe (fMLP), phorbol myristate acetate (PMA) or calcium ionophore (A23187)) elicits a 5-10 fold increase in the surface binding of monoclonal antibodies directed at Mac-1 or p150,95 (Anderson, D.C., et al., J. Infect. Dis. 152:668-689 (1985)) and a 2-3 fold increase in PMNL adhesiveness (Anderson, D.C., et al., J. Clin. Invest. 74:536-551 (1984)). This surface protein "up-regulation" is maximal after approximately 8 minutes incubation at 37°C and is not impeded by inhibitors of protein synthesis. These observations suggest that Mac-1 and p150,95 are translocated from latent intracellular pools to the cell surface by inflammatory stimuli. LAD patients fail to show either increased binding of monoclonal antibodies directed at subunits of the CD18 complex or increased adherence to artificial substrates or endothelium in vitro after exposure to chemotactic factors, PMA or A23187 (Anderson, D.C., et al., J. Clin. Invest. 74:536-551 (1984)). Thus, the profoundly diminished infiltration of PMNL and monocytes into inflamed tissues of LAD patients reflect impaired CD18 expression and the consequent lack of the increased adherence to vascular endothelium that is normally induced by inflammatory stimuli.

## EXAMPLE 12

### Properties of Monoclonal Antibody R6-5-D6

A direct, reliable documentation of CD18-dependent PMNL adherence to HUVEC has been clearly shown in three types of experiments utilizing radiolabeled human neutrophils and HUVEC (Harlan, J.M., et al., Blood 66:167-178 (1985); Pohlman, T.H., et al., J. Immunol. 136:4548-4553 (1986)) or human microvascular endothelium (Tonnesen, M.G., et al., Clin. Res. 34:419a (1986); Tonnesen, M.G., et al., Fed. Proc. 45:379a (1986)):

1) A monoclonal antibody reactive with CD18 (monoclonal antibody 60.3) partially inhibited the adherence of human PMNL to unstimulated HUVEC and HUVEC stimulated with IL-1, LPS and rTNF-α - (Pohlman, T.N., et al., J. Immunol. 136:4548-4553 (1986)).

2) PMNL from patients deficient in CD18 exhibited low levels of adherence and there was no enhancement following stimulation with chemotactic factors (Tonnesen, M.G., et al., Clin. Res. 34:419a (1986); Pohlman, T.N., et al., J. Immunol. 136:4548-4553 (1986)).

3) The augmented adherence following exposure of normal PMNL to a secretagogue (Pohlman, T.N., et al., J. Immunol. 136:4548-4553 (1986)) or the chemotactic factors, C5a, fMLP, LTB$_4$, and PAF (Tonnesen, M.G., et al., Clin. Res. 34:419a (1986); Tonnesen, M.G., et al., Fed. Proc. 45:379a (1986)) was markedly inhibited by anti-β monoclonal antibodies (60.3 and TS1/18) and by 60.1, a monoclonal antibody reactive with CD11b (Wallis, W.J., et al., J. Immunol. 135:2323-2330 (1985); Pohlman, T.N., et al., J. Immunol. 136:4548-4553 (1986)).

## EXAMPLE 13

### CD18-Dependent Adherence of PMNL and HUVEC

Migration of PMNL beneath endothelial cell mono-layers grown on protein-coated glass or plastic substrata has been repeatedly observed (Tonnesen, M. G., et al., J. Clin. Invest. 74:1581-1592; Smedly, L.A., et al., Fed. Proc. 42:386a (1983); Gimbrone, M.A., et al., J. Clin. Invest. 74:1552-1555 (1984); Beesley, J.E., et al., J. Cell. Sci. 38:237-248 (1979); Armstrong, P.B., et al., J. Cell. Biol. 65:439-462 (1975)). This migration occurs in the absence of added chemotactic stimuli and cannot be explained in terms of the relative adhesiveness of the endothelium and substratum (Beesley, J.E., et al., J. Cell. Sci. 38:237-248 (1979)). Since the adherence assay utilized in the present invention allowed direct determination of the percentage of migrating PMNL, this phenomenon could be monitored with each experimental manipulation. Migration of unstimulated PMNL to a position between the HUVEC monolayer and the substratum occurred in a high percentage of PMNL only when the HUVEC monolayer was stimulated with LPS or IL-1. This percentage increased substantially when the adherence assay was performed at 37°C compared to the results at room temperature. A chemokinetic stimulus (i.e., fMLP present in the adherence chamber at a uniform concentration) did not promote this behavior in the PMNL. The migration of unstimulated normal PMNL beneath LPS or IL-1 stimulated HUVEC monolayers was profoundly inhibited by the presence of TS1/18 with the cells throughout the observation period. This result plus the fact that CD18-deficient PMNL exhibited extremely low levels of transendothelial migration implicate a CD18-dependent mechanism. These results are supported by the extremely low adherence-dependent migration of CD18-deficient PMNL (Anderson, D.C., et al., Ann. Rev. Med. 38:175-194 (1987)). R6-5-D6 was equivalent to TS1/18 in blocking PMNL migration beneath the stimulated HUVEC monolayers. In all of these experimental conditions, a monoclonal antibody control (W6/32) of the same isotype as R6-5-D6 failed to produce any change in adherence or migration of PMNL though it bound to the HUVEC surface in greater amounts than R6-5-D6. The observations stated above indicate that the endothelial cells play an active role in the transendothelial migration of PMNL in vitro, and that the determinant recognized by R6-5-D6 is critical to this phenomenon.

## EXAMPLE 14

### Ability of Anti-ICAM-1 Antibody to Inhibit the Migration of Neutrophils into Inflamed Rabbit Lungs

The ability of anti-ICAM-1 antibody to inhibit the migration of neutrophils into inflamed rabbit lungs was studied. One hour after i.v. injection of anti-ICAM-1 antibody R6-5-D6 (at 0.015 mg/kg, 0.15 mg/kg or 1.5 mg/kg), rabbits were injected i.v. with phorbol 12-myristate 13-acetate (PNA) (40 ug/kg), inducing an acute pulmonary inflammatory reaction that peaks at 20-24 hours. The number of neutrophils present at this time

EP 0 314 863 B1

in bronchoalveolar lavage were quantitated using a Coulter Counter and compared to rabbits that received PNA but not anti-ICAM-1. The results of this experiment are shown in Figure 11. The data show that anti-ICAM-1 at 1.5 mg/kg inhibited neutrophil migration 72%.

**EXAMPLE 15**

**Effect of Anti-ICAM-1 Antibody on Myocardial Reperfusion**

In order to determine the effects of anti-ICAM-1 antibody R6-5-D6 on reperfusion injury in rabbit myocardium in vivo, antibody (2.0 mg/kg by i.v. bolus) was administered 15 minutes before one-hour occlusion of the left anterior descending coronary artery, followed by reperfusion of the same artery. The averaged results of nine experiments are shown in Table 7.

## TABLE 7

### Effects of R-6-5 Antibody on Ischemic Damage in Rabbit Myocardium In Vivo

|  | % of Left Ventrical | % of Infarcted Area |  |
|---|---|---|---|
| Area at Risk | 33.8±3.0 | 31.2±6.3 |  |
| % vs.control | +1.0 | −64.0 | p<0.01 |

| Heart rate | before occlusion | end of occlusion | end of reperfusion |
|---|---|---|---|
| Bpm | 263 ± 7 | 269 ± 6 | 238 ± 10 |
| % vs.control | −11.8 | −9.4 | −6.2 |

| Systolic left ventricular pressure | before occlusion | end of occlusion | end of reperfusion |
|---|---|---|---|
| mm Hg | 113 ± 5 | 110 ± 6 | 95 ± 7 |
| % vs.control | +9.5 | +7.6 | +7.5 |

| left ventricular dp/dtmax | before occlusion | end of occlusion | end of reperfusion |
|---|---|---|---|
| mHg/sec | 5.3 ± 0.4 | 4.7 ± 0.2 | 4.0 ± 0.3 |
| % vs.control | −10.2 | −13.0 | +14.0 |

| diastolic aortic pressure | before occlusion | end of occlusion | end of reperfusion |
|---|---|---|---|
| mm Hg | 87 ± 8 | 81 ± 8 | 68 ± 8 |
| % vs.control | −18.0 | −22.5 | −17.1 |

## Claims

1. The use of ICAM-1 or a functional derivative thereof, in the manufacture of a pharmaceutical composition for the treatment of inflammation resulting from a response of the non-specific defense system in a mammalian subject, the functional derivative being:

   A fragment which is a polypeptide subset of ICAM-1 which is capable of binding to a ligand which binds to ICAM-1 and/or when introduced into an appropriate animal will result in the production of antibodies capable of binding to ICAM-1,

   a variant of ICAM-1, being a molecule substantially similar in structure and biological activity to ICAM-1 or said fragment thereof, or

a chemical derivative of any of these.

2. The use according to claim 1 wherein ICAM-1 is used.

3. The use according to claim 1 wherein a functional derivative of ICAM-1 as set forth in claim 1 is used.

4. The use of an antibody capable of binding to ICAM-1, a fragment of an antibody, said fragment being capable of binding to ICAM-1; and a non-immunoglobulin antagonist of ICAM-1 in the manufacture of a pharmaceutical composition for treating inflammation resulting from a response of the non-specific defense system in a mammalian subject.

5. The use according to claim 4 wherein an antibody capable of binding to ICAM-1 is used.

6. The use according to claim 4 wherein a fragment of an antibody, said fragment being capable of binding to ICAM-1, is used.

7. The use according to any one of claims 5 and 6 wherein said antibody is a monoclonal antibody.

8. The use according to claim 7 wherein said monoclonal antibody is the monoclonal antibody R6-5-D6.

9. The use according to claim 4 wherein a non-immunoglobulin antagonist of ICAM-1 is used.

10. The use according to claim 9 wherein said non-immunoglobulin antagonist of ICAM-1 is a non-immunoglobulin antagonist of ICAM-1 other than LFA-1.

11. The use according to any one of claims 1 to 10 wherein said inflammation is associated with a condition selected from the group consisting of: adult respiratory distress syndrome; multiple organ injury syndrome secondary to septicemia; multiple organ injury syndrome secondary to trauma; reperfusion in injury of tissue; acute glomerulonephritis; reactive arthritis; dermatosis with acute inflammatory components; a central nervous system inflammatory disorder; thermal injury; hemodialysis; leukapheresis; ulcerative colitis; Crohn's disease; necrotizing enterocolitis; granulocyte transfusion associated syndrome; and cytokine-induced toxicity.

**Patentansprüche**

1. Verwendung von ICAM-1 oder eines funktionellen Derivates davon zur Herstellung eines pharmazeutischen Mittels zur Behandlung einer Entzündung, die aus einer Antwort des unspezifischen Abwehrsystems in einem Säuger resultiert, wobei das funktionelle Derivat:
   ein Fragment ist, das einen Polypeptid-Teil von ICAM-1 darstellt, das zur Bindung an einen Liganden befähigt ist, der an ICAM-1 bindet, und/oder das nach Einführung in ein geeignetes Lebewesen zur Produktion von Antikörpern führt, die in der Lage sind, an ICAM-1 zu binden,
   eine Variante von ICAM-1 ist, die ein Molekül darstellt, das im wesentlichen ähnliche Struktur und biologische Aktivität wie ICAM-1 oder das Fragment davon aufweist, oder
   ein chemisches Derivat von einem dieser Moleküle ist.

2. Verwendung gemäß Anspruch 1, wobei ICAM-1 verwendet wird.

3. Verwendung gemäß Anspruch 1, wobei ein in Anspruch 1 beschriebenes funktionelles Derivat von ICAM-1 verwendet wird.

4. Verwendung eines Antikörpers, der in der Lage ist, an ICAM-1 zu binden, eines Fragmentes eines Antikörpers, wobei das Fragment in der Lage ist, an ICAM-1 zu binden; und eines Nicht-Immunglobulin-Antagonisten von ICAM-1 zur Herstellung eines pharmazeutischen Mittels zur Behandlung einer Entzündung, die aus einer Antwort des unspezifischen Abwehrsystems in einem Säuger resultiert.

5. Verwendung gemäß Anspruch 4, wobei ein Antikörper verwendet wird, der in der Lage ist, an ICAM-1 zu binden.

**EP 0 314 863 B1**

6. Verwendung gemäß Anspruch 4, wobei ein Fragment eines Antikörpers verwendet wird, wobei das Fragment in der Lage ist, an ICAM-1 zu binden.

7. Verwendung gemäß einem der Ansprüche 5 und 6, wobei der Antikörper ein monoklonaler Antikörper ist.

8. Verwendung gemäß Anspruch 7, wobei der monoklonale Antikörper der monoklonale Antikörper R6-5-D6 ist.

9. Verwendung gemäß Anspruch 4, wobei ein Nicht-Immunglobulin-Antagonist von ICAM-1 verwendet wird.

10. Verwendung gemäß Anspruch 9, wobei der Nicht-Immunglobulin-Antagonist von ICAM-1 ein anderer Nicht-Immunglobulin-Antagonist von ICAM-1 als LFA-1 ist.

11. Verwendung gemäß einem der Ansprüche 1 bis 10, wobei die Entzündung mit einem Zustand assoziiert ist, der ausgewählt ist unter: dem adulten Atemnot-Syndrom; dem multiplen Organverletzungs-Syndrom als Folge einer Sepsis; dem multiplen Organverletzungs-Syndrom als Folge eines Traumas; der Reperfusion bei einer Gewebeverletzung; der akuten Glomerulonephritis; der reaktiven Arthritis; der Dermatose mit akuten entzündlichen Komponenten; einer entzündlichen Erkrankung des zentralen Nervensystems; einer thermischen Verletzung; Hämodialyse; Leukapherese; ulzerativer Kolitis; der Crohn-Krankheit; nekrotisierender Enterokolitis; dem Granulozyten-Transfusion-assoziierten Syndrom; und der Zytokin-induzierten Toxizität.

## Revendications

1. Utilisation de ICAM-1 ou d'un dérivé fonctionnel de celle-ci dans la préparation d'une composition pharmaceutique pour le traitement d'une inflammation résultant d'une réponse du système de défense non spécifique chez un sujet mammifère, le dérivé fonctionnel étant:

un fragment qui est un sous-ensemble polypeptidique de ICAM-1 qui est capable de se lier à un ligand qui se lie à ICAM-1 et/ou qui, lorsqu'il est introduit dans un animal approprié, provoque la production d'anticorps capables de se lier à ICAM-1,

une variante de ICAM-1 qui est une molécule sensiblement semblable, quant à la structure et à l'activité biologique, à ICAM-1 ou audit fragment de celle-ci, ou

un dérivé chimique de l'un quelconque de ceux-ci.

2. Utilisation selon la revendication 1, dans laquelle ICAM-1 est utilisée.

3. Utilisation selon la revendication 1, dans laquelle un dérivé fonctionnel de ICAM-1 selon la revendication 1 est utilisé.

4. Utilisation d'un anticorps capable de se lier à ICAM-1, d'un fragment d'un anticorps, ledit fragment étant capable de se lier à ICAM-1; et d'un antagoniste non-immunoglobuline de ICAM-1 dans la préparation d'une composition pharmaceutique pour traiter une inflammation résultant d'une réponse du système de défense non spécifique chez un sujet mammifère.

5. Utilisation selon la revendication 4, dans laquelle un anticorps capable de se lier à ICAM-1 est utilisé.

6. Utilisation selon la revendication 4, dans laquelle un fragment d'un anticorps est utilisé, ledit fragment étant capable de se lier à ICAM-1.

7. Utilisation selon l'une quelconque des revendications 5 et 6, dans laquelle ledit anticorps est un anticorps monoclonal.

8. Utilisation selon la revendication 7, dans laquelle ledit anticorps monoclonal est l'anticorps monoclonal R6-5-D6.

24

**9.** Utilisation selon la revendication 4, dans laquelle un antagoniste non-immunoglobuline de ICAM-1 est utilisé.

**10.** Utilisation selon la revendication 9, dans laquelle ledit antagoniste non-immunoglobuline de ICAM-1 est un antagoniste non-immunoglobuline de ICAM-1 différent de LFA-1.

**11.** Utilisation selon l'une quelconque des revendications 1 à 10, dans laquelle ladite inflammation est associée à un état choisi dans le groupe consistant en: le syndrome de détresse respiratoire aiguë de l'adulte, le syndrome de lésions d'organes multiples consécutif à une septicémie, le syndrome de lésions d'organes multiples consécutif à un traumatisme, la reperfusion dans la lésion de tissus, la glomérulonéphrite aiguë, l'arthrite réactive, la dermatose à composantes inflammatoires aiguës, un trouble inflammatoire du système nerveux central, une lésion thermique, l'hémodialyse, la leucophérèse, la colite ulcéreuse, la maladie de Crohn, l'entérocolite nécrosante, le syndrome associé à une transfusion de granulocytes et la toxicité induite par une cytokine.

FIGURE 1

EP 0 314 863 B1

FIGURE 2

% ADHERENCE

Hours after addition of stimulant

◇ LPS

● IL-1

FIGURE 3

FIGURE 4

PMNL:

PBS

PBS

TS1/18

fMLP

OKM1
fMLP,
TS1/18

TS1/22

R6-5-D6

PBS

TS1/18

PERCENT ADHERENCE

10 20 30 40 50 60 70 80 90 100

(9)

** (11)

** (4)

(4)

(4)

(3)

(4)

(9)

** (3)

(3)

FIGURE 5

EP 0 314 863 B1

FIGURE 6

FIGURE 7

FIGURE 8

FIGURE 9

EP 0 314 863 B1

FIGURE 10

FIGURE 11

FIGURE 11. THE EFFECT OF ANTI-ICAM-1 MONOCLONAL ANTIBODY ON
RABBIT LUNG NEUTROPHIL INFLUX

DOSE OF ANTI-ADHESION MAb (mg/kg):

36